# EUROPÄISCHE PATENTSCHRIFT

(11) **EP 1 913 140 B1**
(45) Veröffentlichungstag und Bekanntmachung des Hinweises auf die Patenterteilung: **24.12.2008**
(21) Anmeldenummer: 06763003.8
(22) Anmeldetag: 07.08.2006
(51) Int. Cl.: C12N 15/10, C40B 10/00

(54) **VERFAHREN FÜR DIE KONTINUIERLICHE ZIELGERICHTETE EVOLUTION VON PROTEINEN IN VITRO**
METHOD FOR CARRYING OUT THE SELECTIVE EVOLUTION OF PROTEINS IN VITRO
PROCEDE POUR L'EVOLUTION CIBLEE CONTINUE DE PROTEINES IN VITRO

(30) Priorität: 08.08.2005 DE 102005037351
(43) Veröffentlichungstag der Anmeldung: 23.04.2008
(73) Patentinhaber: GENEART AG, 93053 Regensburg (DE)
(72) Erfinder: LISS, Michael, 93047 Regensburg (DE)
(74) Vertreter: Weiss, Wolfgang
(86) Internationale Anmeldenummer: PCT/EP2006/007798
(87) Internationale Veröffentlichungsnummer: WO 2007/017229

(56) Entgegenhaltungen:
- WO-A-20/05030957
- BREAKER RONALD R ET AL: "Continuous in vitro evolution of bacteriophage RNA polymerase promoters" BIOCHEMISTRY, Bd. 33, Nr. 39, 1994, Seiten 11980-11986, XP002416112 ISSN: 0006-2960
- PELLETIER ET AL: "An in vivo library-versus-library selection of optimized protein-protein interactions" NATURE BIOTECHNOLOGY, NATURE PUBLISHING GROUP, NEW YORK, NY, US, Bd. 17, Juli 1999 (1999-07), Seiten 6836-90, XP002156488 ISSN: 1087-0156

## Beschreibung

Die vorliegende Erfindung betrifft die Herstellung von Varianten eines Proteins in einem *in vitro* Evolutionsverfahren.

### Hintergrund der Erfindung

Die zunehmende Bedeutung der Biotechnologie in den Bereichen der Medizin, der chemischen Industrie und der Agrarwirtschaft verlangt zunehmend nach Proteinen, die für ihren jeweiligen Einsatzzweck optimal angepasst sind. Zunächst werden diese Proteine hauptsächlich aus der Umwelt, meist im Rahmen von so genannten Metagenom-Screenings isoliert. Vermehrt werden sie in der Folge über unterschiedliche Methoden an die geplanten "artifiziellen" Einsatzbedingungen angepasst.

So werden beispielsweise Enzyme benötigt, die hitzestabiler sind als ihre natürlichen Varianten, eine andere Substratspezifität haben oder höhere Aktivitäten zeigen. Pharmazeutische Proteine etwa sollen längere Halbwertszeiten aufweisen, um geringer dosiert werden zu können, oder über die hochaffine und spezifische Bindung an Zielmoleküle, krankheitsassoziierte Stoffwechsel- oder Infektionswege inhibieren.

### Stand der Technik

Diese Anpassung geschieht zum Teil über rationale Protein Design Ansätze. Bislang gibt es jedoch nur sehr beschränkte Möglichkeiten, von der gewünschten Funktion - dem Phänotyp - eines Proteins auf dessen Struktur zu schließen bzw. von der dreidimensionalen Struktur des Proteins die entsprechende Primärsequenz abzuleiten. Um dennoch eine Funktionssteigerung eines Proteins zu erreichen, bedient man sich derzeit teilevolutionärer Ansätze, die unter dem Begriff der "zielgerichteten Evolution" (Directed Evolution) zusammengefasst werden.

Nach dem derzeitigen Stand der Technik beruhen die bisherigen Verfahren der Directed Evolution im Wesentlichen auf der Generierung einer Vielzahl von Varianten (Nachkommen) des zu verbessernden Proteins und deren Selektion nach verbesserten Abkömmlingen. Dabei kann die Zahl der untersuchten Mutanten z.T. sehr groß sein, liegt jedoch in der Regel bei unter 10¹¹. Betrachtet man ein Protein von nur 100 Aminosäuren, so existieren theoretisch 20¹⁰⁰ = 10¹³⁰ unterschiedliche Varianten davon. Eine Bibliothek einer Größe von 10¹¹ deckt demnach nur einen sehr kleinen Bruchteil der möglichen Varianten ab. Die Wahrscheinlichkeit, in einer solchen Bibliothek die theoretisch beste Variante zu finden, ist annähernd null.

Um eine große Anzahl von Varianten auf maximale Affinität zu einem Zielmolekül hin zu screenen, wurde bereits eine Vielzahl von Protokollen entwickelt (z.B. Yeast Two-Hybrid, Bacterial Display, Phage Display, Ribosomal Display, mRNA Display).

Für das Screenen anderer Eigenschaften, wie etwa enzymatische Aktivität, werden meist Assay-Formate benötigt, die die Untersuchung von nur einer verhältnismäßig kleinen Anzahl von Varianten (<10⁶) erlauben. Diesen Methoden gemeinsam ist, dass sie auf die Generierung einer Bibliothek von Mutanten sowie deren anschließende Selektion beschränkt sind. Eine Replikation (= nächste Generation von Mutanten des "Gewinners" der zuletzt erfolgten Selektion) ist mit diesen Protokollen zwar manuell möglich, jedoch ebenso aufwändig wie der vorangegangene Schritt. Daher erstrecken sich entsprechende Ansätze in aller Regel nur über ein bis zwei Generationen. Aus diesem Grund handelt es sich bei den erwähnten Protokollen nicht um evolutionäre Ansätze im eigentlichen Sinne, sondern vielmehr um die ausschließliche Selektion vorhandener Variantenpools. Folglich kann das Potenzial der schrittweisen Adaption über viele Generationen hinweg nicht genutzt werden, was jedoch die notwendige Voraussetzung dafür wäre, unter Umständen die aktivste Variante aus einer astronomisch großen Anzahl von Möglichkeiten zu identifizieren.

Das Prinzip der Evolution mit seinen drei Voraussetzungen - Replikation, Mutation und Selektion - vermag innerhalb eines gegebenen Systems eine gerichtete Entwicklung von einfachen bis hin zu hoch angepassten Strukturen zu bewirken. Dieses Modell des so genannten "blinden Uhrmachers" ermöglicht die Schaffung von Komplexität ohne ein planerisches Zutun und ohne notwendige Kenntnis von Strukturdaten.

1989 konnte von Bauer et al. (PNAS (1989) 86, 7937-7941) gezeigt werden, dass in einer Kapillare, in der sich Qβ-Replikase befindet und die an einem Ende mit beliebiger RNA angeimpft wird, ein kontinuierlicher evolutionärer Prozess stattfindet. Es entsteht eine Polymerisationsfront entlang der Kapillare, in deren Verlauf die entstehenden RNA-Polymere hin zu phagenspezifischen Sequenzen/Sekundärstrukturen evolvieren, da diese von der Replikase schneller repliziert werden. Obwohl dieses Experiment keinen unmittelbaren praktischen Nutzen aufweist, belegt es doch deutlich das Potential der drei Faktoren Replikation, Mutation und Selektion.

WO 02/22869 beschreibt Verfahren zur Verwendung bei der *in vitro* Evolution von Molekülbibliotheken. Hierbei wird das "Two Hybrid"-System verwendet. Es wird allerdings hier lediglich eine Polymerase verwendet, die eine erhöhte Fehlerfrequenz hat, jedoch keine Reverse Transkriptase. Dieses Dokument betrifft somit die so genannte "Error-Prone-PCR".

WO 2004/024917 beschreibt ein Verfahren zur gerichteten Evolution von Enzymen, wobei das Protein und dessen kodierende DNA räumlich gekoppelt und in einem Kompartiment eingeschlossen sind. Das Protein liegt als Fusionskonstrukt mit einem Peptid-Tag vor. Ausgangsmaterial ist eine DNA-Bibliothek. Es werden jedoch keine Ligand-Protein-Wechselwirkungen zur Selektion eingesetzt und keine Mutationen durch RNA-Polymerase eingeführt.

WO 2005/030957 offenbart eine *in vitro* Selektion von Proteinen, die als Fusionsproteine an kodierende DNA gekoppelt sind.

Ein ähnliches System ist auch bei Bernath, K. et al. beschrieben (J. Mol. Biol. (04.02.2005) 345 (5), 1015-1026).

WO 01/51663 offenbart integrierte Systeme und Verfahren für die Modifikation von Nukleinsäuren. Insbesondere wird hier das NASBA-Verfahren unter Verwendung von Qß-Replikase eingesetzt. Ein Hinweis auf die Einführung von Mutationen durch RNA-Polymerase ist jedoch nicht offenbart.

Obgleich Fusionsproteine aus Reverser Transkriptase und anderen Proteinen bereits bekannt sind, gibt es keinen Hinweis auf die Verwendung von RT-Fusionsproteinen oder T7-RNA-Polymerase-Fusionsproteinen in der *in vitro* Evolution.

Auch Qß-Replikasesysteme sind bereits eingehend untersucht worden (McCaskill und Bauer (Proc. Natl. Acad. Sci. USA 1993, 90, 4191-4195). Diese Publikation beschreibt Evolutionswellen in einem Qß-Replikasesystem. Die RNA-Front wandert um so schneller, je höher die "Fitness" der Replikons ist. Das erfindungsgemäße System ist dieser Publikation jedoch nicht zu entnehmen.

WO 2004/108926 offenbart die künstliche Evolution von Proteinen mit verbesserter Bindungsfähigkeit. Die Proteine werden in RNA-Replikons kodiert, die durch fehlerhafte Replikation eine Quasi-Spezies bilden. Es handelt sich hierbei jedoch um eine *in vivo* Expression.

Bisher gibt es noch keine *in vitro* Systeme, welche es möglich machen, Proteine zu evolvieren. Es besteht immer noch ein Bedarf an Systemen und Verfahren zur zielgerichteten Evolution von Proteinen mit verbesserten Eigenschaften, welche das aufwändige Erstellen und Screenen von Mutanten-Bibliotheken vermeidet.

Gegenstand der vorliegenden Erfindung ist ein Verfahren zur Herstellung von Varianten Y' eines Proteins Y, umfassend die Schritte:
(A) Bereitstellen eines *in vitro*-Expressionssystems, umfassend
   (i) eine Nukleinsäuresequenz S, die für ein zu variierendes Protein Y kodiert,
   (ii) ein Zielmolekül X, welches in der Lage ist, an das Protein Y und/oder mindestens eine Variante Y' davon zu binden,
   (iii) eine RNA-Polymerase (Pol), welche in der Lage ist, die Nukleinsäuresequenz S zu transkribieren,
   (iv) eine Reverse Transkriptase (RT), welche in der Lage ist, Transkripte der Nukleinsäuresequenz S revers zu transkribieren,
   wobei entweder das Zielmolekül X an Pol und das Protein Y an RT gekoppelt ist,
   oder das Zielmolekül X an RT und das Protein Y an Pol gekoppelt ist,
(B) Inkubieren des *in vitro* Expressionssystems aus (A) unter Bedingungen, welche eine Transkription, eine Reverse Transkription und eine Translation unter Bildung von Varianten Y' des Proteins Y und dafür kodierender Nukleinsäuresequenzen S' ermöglichen, und welche die Bildung von Varianten Y' mit verbesserten Bindungseigenschaften für das Zielmolekül X begünstigen,
(C) Isolieren und gegebenenfalls Charakterisieren derjenigen Varianten Y', welche verbesserte Bindungseigenschaften für die Bindung an X aufweisen und/oder Isolieren von für Y' kodierenden Nukleinsäuresequenzvarianten S'.

Insbesondere betrifft die Erfindung ein Verfahren zur Herstellung von Varianten Y' eines Proteins Y umfassend die Schritte:
(A) Bereitstellen eines in vitro-Expressionssystems, umfassend
   (a) eine Nukleinsäuresequenz S, kodierend für
      (a1) eine durch eine Polymerase (Pol) *in trans* aktivierbare Transkriptionskontrollsequenz,
      (a2) ein zu variierendes Protein Y, und
      (a3) eine Reverse Transkriptase (RT),
         oder alternativ
      (a3') eine Polymerase (Pol),
         wobei die für (a2) und (a3) bzw. (a3') kodierenden Sequenzabschnitte für ein Fusionsprotein unter der Kontrolle der *in trans* aktivierbaren Transkriptionskontrollsequenz von (a1) kodieren,
   (b) einen Proteinkomplex, umfassend
      (b1) eine Komponente X, welche in der Lage ist, an mindestens eine Variante des zu variierenden Proteins Y zu binden, und
      (b2) die RNA-Polymerase Pol zur Transkription der Nukleinsäuresequenz S aus (a)
         oder alternativ
      (b2') die Reverse Transkriptase RT,
(B) Inkubieren des *in vitro* Expressionssystems aus (A) unter Bedingungen, welche die Bildung von Varianten Y' des Proteins Y ermöglichen,
(C) Isolieren und gegebenenfalls Charakterisieren derjenigen Varianten Y', welche verbesserte Bindungseigenschaften für die Bindung an X aufweisen und/oder Isolieren der für Y' kodierenden Nukleinsäuresequenzvarianten S'.

Inhalt dieser Erfindung sind somit autonome Systeme, die einerseits die Selektion einer gegebenen Variantenbibliothek im Labor zulassen und gleichzeitig einen Replikationsmechanismus beinhalten. Dabei soll die Selektion - wie bei der natürlichen Evolution - über eine bevorzugte Replikation besser angepasster Varianten realisiert werden.

Die vorliegende Erfindung stellt somit ein Verfahren bereit, welches die Evolution von Proteinen mit verbesserten Eigenschaften, insbesondere mit verbesserten Bindeeigenschaften, ermöglicht. Das erfindungsgemäße System kombiniert eine *in vitro*-Transkription mit einer *in vitro*-Translation und Reverser Transkription. Überraschenderweise hat sich herausgestellt, dass es möglich ist, in einem *in vitro* System diese drei Vorgänge zu einem natürlichen Evolutionsverfahren zu kombinieren. In diesem System werden mRNA-Transkripte einer Nukleinsäuresequenz kodierend für ein zu variierenden Proteins generiert, welche Transkripte anschließend durch Reverse Transkription wieder in cDNAs umgeschrieben werden, die dann erneut transkribiert und revers transkribiert werden können.

Diese Art der Amplifikation unter Verwendung von RNA-Polymerase und Reverser Transkriptase ist bereits bekannt als Alternative zur PCR. Eines dieser bereits bekannten Nukleinsäure-Amplifikationsverfahren ist das NASBA-Prinzip (siehe z.B. Romano et al., 1995, J. Virol. Methods, 54 (2-3): 109-119; Romano et al., 1997, Immunol. Invest. 26 (1-2):15-28).

Vorzugsweise durch den reversen Transkriptionsschritt mit Reverser Transkriptase, welche bekanntlich aufgrund des Fehlens einer Proofreading-Funktion eine gewisse Fehlerrate besitzt, werden ausgehend von den Transkripten cDNAs erzeugt, von denen sich zumindest einige von dem ursprünglichen Template durch Mutationen unterscheiden. Werden diese cDNAs wiederholt transkribiert und revers transkribiert, so erhält man eine Vielzahl von Varianten auf Nukleinsäureebene, welche für Varianten des zu variierenden Protein kodieren.

Im erfindungsgemäßen *in vitro* System werden die Transkripte unter Bildung von Proteinen und Varianten des ursprünglich kodierten Proteins translatiert.

In dem erfindungsgemäßen Expressionssystem sind in einem definierten Raum die Reagenzien, die für die Transkription, Reverse Transkription und Translation benötigt werden (wie etwa Primer, dNTPs, NTPs, tRNAs, Aminosäuren etc.) in ausreichender Menge vorhanden. Wenn in dem definierten Raum an einer bestimmten Stelle die Nukleinsäuresequenz S, das Zielmolekül X, die RNA-Polymerase, und die Reverse Transkriptase bereitgestellt werden, z.B. durch Animpfen, so werden bei Ablauf der Transkription, Reversen Transkription und Translation an der Animpfstelle die entsprechenden Reagenzien aufgebraucht, und es bildet sich eine fortschreitende so genannte Reaktionsfront, an welcher sich diejenigen Transkripte, Proteine und reverse Transkripte (cDNAs) befinden, welche zuletzt gebildet wurden.

Das verwendete Replikationssystem ist vorzugsweise so aufgebaut, dass es eine ausreichende Mutationsrate während der Replikationen erlaubt. Die Anzahl der auf diese Weise theoretisch getesteten Varianten eines Ausgangskonstrukts errechnet sich aus der Anzahl der Nachkommen eines Gewinners einer Generation zur Potenz der Gesamtanzahl der Generationen eines Experiments (z.B. 10 Nachkommen pro Generation bei 400 Generationen = 10⁴⁰⁰ mögliche Varianten.) Zwar ist es unmöglich, dass jede dieser 10⁴⁰⁰ einzelnen Varianten explizit in einem Experiment physikalisch vorhanden ist, jedoch sucht sich das System selbst einen "Pfad" innerhalb eines komplexen virtuellen Terrains, der stets hinauf bis zum absoluten Maximum führt. Nur die Varianten entlang des Pfades haben während des Experiments existiert, theoretisch möglich sind alle Punkte (Varianten) des Terrains.

Die Erfinder der vorliegenden Erfindung haben Möglichkeiten gefunden, die Bildung der Proteinvarianten so zu steuern, dass die Transkription, Reverse Transkription und Translation von bestimmten der gebildeten mutierten Nukleinsäuren, die für Proteinvarianten mit verbesserten Eigenschaften kodieren, bevorzugt abläuft. Dadurch sind derartige cDNAs und Transkripte, die für verbesserte Proteinvarianten kodieren, in größerer Anzahl vorhanden und bewegen sich schneller an der Polymerisationsfront fort.

Der Evolutionsdruck, der für die Bildung von ganz bestimmten, verbesserten Varianten notwendig ist, wird wie folgt erzeugt.

Das zu variierende Protein Y ist in der Lage, an ein Zielmolekül X zu binden. Variiert man das Protein Y im Sinne der vorliegenden Erfindung, so entstehen Varianten Y', von denen zumindest einige verbesserte Bindungseigenschaften für die Bindung an das Zielmolekül X aufweisen können. Um die Erzeugung solcher Varianten zu begünstigen und gegebenenfalls diese Varianten noch weiter zu variieren, um die Bindungseigenschaften noch mehr zu verbessern, werden die Bedingungen in dem erfindungsgemäßen Verfahren so gewählt, dass die Bindung zwischen X und der Variante Y' mit verbesserten Bindungseigenschaften dazu führt, dass diejenigen Transkripte, die für die Varianten Y' mit verbesserten Bindungseigenschaften kodieren, bevorzugt revers transkribiert werden. Dadurch entstehen cDNAs, welche für die Varianten Y' mit verbesserten Bindungseigenschaften kodieren. Dadurch, dass die Reverse Transkription von Transkripten für Varianten Y' mit verbesserten Bindungseigenschaften begünstigt wird, wird auch die erneute Transkription und Translation der Varianten quantitativ begünstigt.

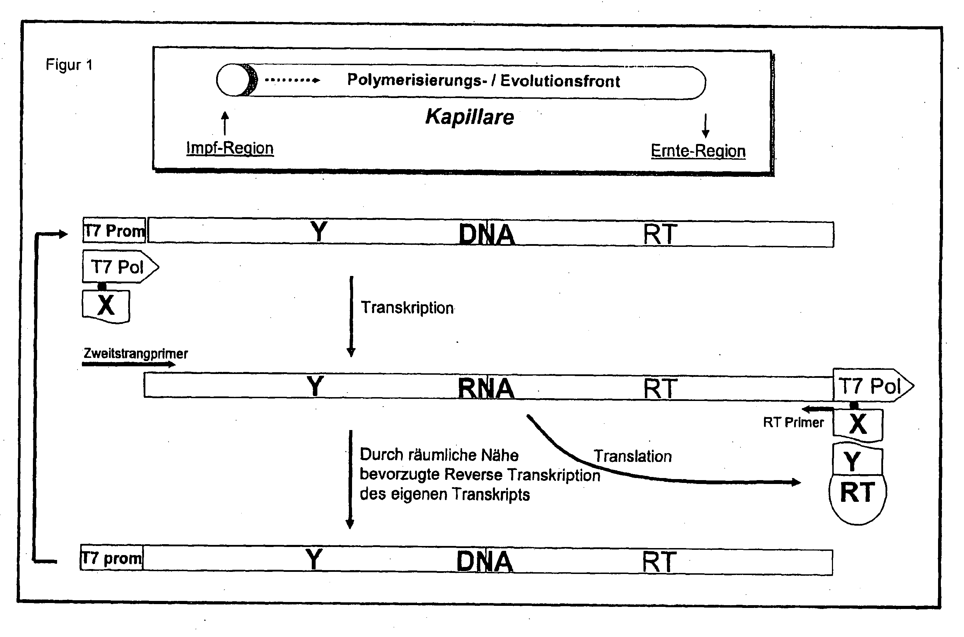

Um dies zu erreichen, wird vorzugsweise ein Komplex aus RNA-Polymerase, Reverser Transkriptase, X und einer Variante Y' gebildet, der eine reverse Transkription desjenigen Transkripts ermöglicht, von welchem diese Variante Y' kodiert wurde. Aufgrund der räumlichen Nähe der RT zu dem Transkript, welches für eine Variante Y' mit verbesserten Bindungseigenschaften an X kodiert, wird dieses Transkript von derselben RT revers transkribiert, die mit der verbesserten Variante Y' komplexiert ist (oder mit dieser ein Fusionsprotein bildet). Figur 1 zeigt eine erfindungsgemäße Alternative des Verfahrens schematisch.

Vorzugsweise wird in dem erfindungsgemäßen Verfahren Y von einer Nukleinsäure S als Fusionsprotein mit einem Protein P kodiert, wobei das Protein P ein Protein ist, welches an der Transkription oder der Reversen Transkription beteiligt ist. P kann somit eine RNA-Polymerase (Pol) oder eine Reverse Transkriptase (RT) sein, oder es kann ein Protein sein, welches mit einer RNA-Polymerase oder einer Reverse Transkriptase assoziiert ist oder an selbige gebunden werden kann.

In zwei bevorzugten Alternativen (1 und 2) des erfindungsgemäßen Verfahrens wird das Protein Y entweder (1) mit RT assoziiert oder als Fusionsprotein mit RT kodiert oder (2) mit Pol assoziiert oder als Fusionsprotein mit Pol kodiert.

Vorzugsweise wird Y als Fusionsprotein von der Nukleinsäuresequenz S kodiert. Es kann aber auch als Protein Y von der Nukleinsäuresequenz S kodiert werden und nach der Translation mit der entsprechenden weiteren Komponente assoziierten. Dies kann über Protein-Interaktionen oder über Bindemoleküle (z.B. Biotin/Avidin, Biotin Streptavidin etc.) erreicht werden. Weitere Möglichkeiten der Kopplung von Y bzw. Y' an RT oder Pol beinhalten u.a. eine chemische Kopplung über kovalente Verknüpfung oder auch eine Verknüpfung über quervernetzende Moleküle, z.B. so genannte Linker, z.B. bifunktionelle Cross-Linker. Die hierbei geeigneten Verknüpfungsreagenzien können vom Fachmann auf dem Gebiet problemlos ausgewählt werden.

In einer Variante zur ersten Alternative des erfindungsgemäßen Verfahrens kann der Komplex aus Protein Y und RT auch von zwei verschiedenen Nukleinsäuresequenzen S1 und S2, jeweils unter der Kontrolle einer geeigneten Transkriptionskontrollsequenz, kodiert werden, wobei in diesem Fall kein Fusionsprotein entsteht, sondern eine Bindung zwischen Y und RT auf andere Art und Weise bewirkt werden kann, beispielsweise über Biotin/Avidin oder Streptavidin. Das heißt, die Nukleinsäuresequenz S liegt in diesem Fall in Form von zwei Nukleinsäuresequenzen S1 und S2 vor. Wichtig ist, dass am Ende der Translation das zu variierende Protein Y an ein RT-Protein gebunden oder damit komplexiert vorliegt.

Das Zielmolekül X ist entsprechend den genannten Alternativen entweder (1) mit Pol oder (2) mit RT assoziiert oder bildet ein Fusionsprotein mit den jeweiligen Komponenten.

Das Zielmolekül X kann ein Protein, ein Peptid oder auch eine Nukleinsäure oder ein anderes Molekül sein. Es kann somit entweder als Nukleinsäure (z.B. kodiert auf einem Plasmid) bereitgestellt werden und im erfindungsgemäßen Expressionssystem exprimiert werden, oder es kann als Molekül bereitgestellt werden.

In der ersten Alternative wird ein zu variierendes Protein Y in Form eines Fusionsproteins mit einer Reversen Transkriptase von einer Expressionskassette kodiert. Die Expressionskassette wird in Form einer Nukleinsäuresequenz S bereitgestellt, welche im Verlauf des erfindungsgemäßen Evolutionsverfahrens transkribiert und translatiert wird. Zusätzlich zur Transkription und reversen Transkription erlaubt das vorliegende Verfahren eine Translation des Transkripts der Nukleinsäure S. Dadurch entsteht in der ersten Alternative ein Fusionsprotein, welches das zu variierende Protein Y sowie die Reverse Transkriptase RT umfasst.

Die Reverse Transkriptase kann aber auch gemäß der zweiten Alternative als Komplex mit X bereitgestellt werden, entweder ebenfalls als Fusionsprotein oder als Komplex, bei dem RT auf andere Art und Weise an X gekoppelt ist. Gemäß der zweiten Alternative kann das Fusionsprotein, das von der Nukleinsäure S kodiert wird, statt RT die RNA-Polymerase Pol umfassen. In diesem Fall muss zu Beginn der Reaktion eine RNA-Polymerase bereitgestellt werden. Wird das Verfahren dann unter Bedingungen durchgeführt, die eine Transkription und eine Translation erlauben, so kann die durch Translation generierte, von S kodierte Polymerase Pol in folgenden Zyklen die Ausgangsnukleinsäure S sowie gegebenenfalls Varianten S' davon als Template für eine Transkription verwenden.

Die Transkriptionskontrollsequenz ist vorzugsweise ein Promotor, der aus allen gängigen, für RNA-Polymerisationsreaktionen geeigneten Promotoren ausgewählt werden kann. Bevorzugt sind für die Zwecke der vorliegenden Erfindung der T7-, T3- und der SP6-RNA-Polymerasepromotor. Es können jedoch auch andere Promotoren ausgewählt werden.

Zur Durchführung der Transkription wird dem entsprechend eine RNA-Polymerase bereitgestellt, vorzugsweise ausgewählt aus RNA-Polymerase T7, RNA-Polymerase T3 und RNA-Polymerase SP6. Die RNA-Polymerase kann von einer Nukleinsäure kodiert vorliegen, oder sie kann als Protein (oder Fusionsprotein oder Komplex) in das Expressionssystem eingebracht werden, je nach der ausgewählten Alternative des erfindungsgemäßen Verfahrens.

Wichtig ist für die Zwecke der vorliegenden Erfindung, dass, wenn eine Bindung zwischen X und Y oder X und Y' zustande kommt, durch diese Bindung die Reverse Transkriptase RT in räumliche Nähe des Transkripts gebracht wird. Das heißt, es entsteht ein räumlicher Komplex aus P/X/(Y oder Y')/RT. Das bedeutet, dass nach dem Ablauf einer Transkriptionsreaktion und einer Translationsreaktion soeben transkribierte mRNA-Moleküle, die für Y' kodieren, in räumlicher Nähe zu der RNA-Polymerase Pol vorliegen, welche mit dem Protein X gekoppelt ist. Findet nun eine Bindung zwischen dem Protein X und Y oder Y' statt, wobei Y bzw. Y' komplexiert mit RT vorliegt, so befindet sich vorzugsweise ein RT-Protein in direkter räumlicher Nähe zu dem Transkript. Durch die räumliche Nähe zwischen dem RNA-Transkript und dem Protein RT über Y oder Y' wird eine Reverse Transkription des Transkripts für Y' gefördert.

Es können somit Varianten der Nukleinsäuresequenz S auf mRNA-Ebene generiert werden, welche dann zu Translationsprodukten führen, welche ebenfalls Varianten darstellen. Auf diese Weise können Varianten des zu variierenden Proteins Y, nämlich Varianten Y' entstehen. Dabei entstehen Varianten, welche besser oder welche schlechter an das Protein X binden.

Der Evolutionsvorteil für Varianten Y', welche bessere Eigenschaften in Bezug auf die Bindung an Protein X aufweisen, liegt in einer bevorzugten Ausführungsform darin, dass sich kurz nach der Transkription eine funktionelle RT in unmittelbarer Nähe zu ihrem eigenen Transkript und damit dann für Y' befindet und dieses bevorzugt revers transkribiert. Dadurch entstehen schneller und/oder mehr cDNAs, welche für eine verbesserte Variante Y' kodieren.

Diese Varianten mit Selektionsvorteil dienen dann wiederum als Ausgangsnukleinsäuresequenzen S', welche wiederum transkribiert, revers transkribiert und translatiert werden kann. Auf diese Weise entsteht eine bevorzugte und damit verstärkte Generierung von Nukleinsäuresequenzvarianten S' und Varianten Y' des Proteins Y, welche nach Ablauf des Verfahrens nach einem angemessenen Zeitraum isoliert werden können. In gleicher Weise kann auch die Fehlerrate der RT bei der Generierung von Varianten der Reversen Transkriptase ausgenutzt werden.

Diesen Effekt macht sich das System der vorliegenden Erfindung zu Nutze, indem es durch die Auswahl der Polymerase und/oder Reversen Transkriptase und/oder der Bedingungen Varianten S' der ursprünglich bereitgestellten Nukleinsäuresequenz S und damit Varianten Y' der davon kodierten Proteine, insbesondere Protein Y, erzeugt.

Vorzugsweise werden DNA-abhängige RNA-Polymerasen verwendet, welche eine bestimmte Fehlerrate aufweisen, wodurch Transkripte mit Mutationen entstehen. Diese Mutationen können beispielsweise Punktmutationen sein, es können z.B. Substitutionen, Deletionen oder Insertionen durch die Polymerase erzeugt werden.

Sowohl RNA-Polymerasen , als auch RT haben keine Proofreading-Funktion und besitzen somit eine höhere Mutationsrate als Polymerasen mit Proofreading-Funktion.

Aus diesem Grund werden daher vorzugsweise Polymerasen verwendet, welche eine höhere Fehlerrate als die korrespondierenden Wildtyp-Polymerasen aufweisen. Es sind im Stand der Technik bereits Polymerasen bekannt, welche eine erhöhte Mutationsrate aufweisen. Bevorzugt sind die T7-Polymerase und T3-Polymerase, aber auch SP6-Polymerase. Von T7-Polymerase existieren bereits Varianten mit erhöhten Fehlerraten (Brakmann und Grzeszik, 2001, Chem. Biochem. 2, 212-219).

In gleicher Weise kann auch eine Reverse Transkriptase verwendet werden, welche in der Lage ist, Transkripte mit einer gewissen Mutationsrate zu generieren.

Vorzugsweise werden für Pol und RT Polymerasen bzw. Reverse Transkriptasen ohne 5'-3'-Exonukleaseaktivität eingesetzt. Für die Zwecke der vorliegenden Erfindung kann entweder die Fehlerrate der RNA-Polymerase oder der Reversen Transkriptase ausgenutzt werden oder auch beides.

Es ist auch möglich, zur Generierung von Transkripten oder/und reversen Transkripten alternativ oder ergänzend zu den genannten Pol- und RT-Molekülen mit erhöhter Fehlerrate die Mutationsrate (Fehlerrate) auf andere Weise zu erhöhen. Beispielsweise können mutagene Agenzien, Nukleotidanaloga als Substrate für Pol und/oder RT oder/und auch z.B. UV-Strahlung eingesetzt werden. Der Fachmann kann solche mutagenen Substanzen selbst auswählen, da sie im Stand der Technik bekannt sind.

Das erfindungsgemäße Verfahren ist geeignet für eine Durchführung *in vitro*. Die Expressionsumgebung kann jede hierfür geeignete Expressionsumgebung sein, vorzugsweise wird eine Kapillare verwendet. Anstelle einer Kapillare ist es jedoch auch möglich, eine zweidimensionale Expressionsumgebung zu wählen, beispielsweise eine Umgebung zwischen zwei Glasplatten. Es kann auch eine dreidimensionale Expressionsumgebung verwendet werden.

Wird eine Kapillare verwendet, so wird diese an einem Ende an der so genannten Impf-Region mit einer Nukleinsäuresequenz S angeimpft.

Wird ein zweidimensionales System verwendet, so ist es möglich, das System an einer Ecke oder auch an einer anderen Stelle, z.B. in der Mitte, mit der Nukleinsäuresequenz S anzuimpfen. Durch ein solches zweidimensionales System erhöhen sich die Freiheitsgrade und die Anzahl der Pfade, welche die Evolution des zu variierenden Proteins Y einschlägt. Kontrollierbar bleibt der Prozess in beiden Fällen über die Beobachtung der Polymerisationsfronten. Dies kann beispielsweise durch Markierungsreagenzien geschehen, wie zum Beispiel interkalierende Reagenzien. Neue Varianten bilden sich dann als schnellere, sich linear im Kapillarsystem oder kreisförmig im zweidimensionalen System ausbreitende Front. Die Probenentnahme bzw. die Isolierung der gewünschten Varianten kann dann am Ende der Kapillaren oder am Rand eines zweidimensionalen Systems oder an beliebiger anderer Stelle geschehen.

Es ist auch möglich, ein größervolumiges dreidimensionales Expressionssystem anzuwenden. Hierfür sollten dann die Expressionsumgebungsbedingungen so gewählt werden, dass das Reaktionsmedium viskoser beschaffen ist, da die Flüssigkeit in einem größervolumigen System weniger durch Kapillarkräfte stabilisiert wird.

Die Geschwindigkeit der Polymerisationsfront entlang der Kapillare oder zwei- oder dreidimensionalen Systems kann als Indikator für das Voranschreiten der Entstehung von Varianten und damit der Verbesserung der Bindungseigenschaften des zu variierenden Proteins Y und dessen Varianten Y' dienen.

Des Weiteren werden alle notwendigen Reaktionsbedingungen für eine Transkription und Translation entsprechend eingestellt. Insbesondere gehört hierzu die Bereitstellung von entsprechenden Oligonukleotiden als Primer, und von Nukleotiden, insbesondere dNTPs, NTPs etc. Für die Translation werden entsprechende Enzyme und Reagenzien benötigt, wie etwa Ribosomen, tRNAs, Aminosäuren, Energieträger (wie etwa GTP und dgl.) etc.

Derartige Reaktionsbedingungen können vom Fachmann problemlos eingestellt werden und es können auch die entsprechenden Primer-Oligonukleotide vom Fachmann ausgewählt werden.

Das erfindungsgemäße Verfahren erlaubt die Generierung von Varianten Y' des Proteins Y in dem bereitgestellten System von selbst. Es ist somit möglich, zu variierende Proteine Y sich selbst in eine bestimmte Richtung entwickeln zu lassen, die man durch die Bindefähigkeit der Varianten Y' an das Zielmolekül X steuern kann.

Es ist somit möglich, jedes beliebige Protein Y in dem erfindungsgemäßen Verfahren zu evolvieren. Es wird hierzu einfach ein entsprechendes Zielprotein X ausgewählt, das in der Lage ist, an Y zu binden, und man erhält ohne aufwändiges Screening Varianten mit verbesserten Bindeeigenschaften für X.

Vorzugsweise werden die Nukleinsäuresequenz-Varianten S' (entweder RNA oder DNA oder beides) an der Reaktionsfront isoliert. Man kann aber auch die Y' isolieren.

Weiterhin betrifft die vorliegende Erfindung einen Kit zur Herstellung eines Proteins mit verbesserten Eigenschaften, umfassend
(a) eine Expressionsumgebung,
(b) eine Nukleinsäuresequenz S, kodierend für eine durch eine RNA-Polymerase Pol *in trans* aktivierbare Expressionskontrollsequenz, ein zu variierendes Protein Y, eine Reverse Transkriptase RT,
(c) einen Komplex, umfassend Pol und ein Protein X, welches in der Lage ist, an mindestens eine Variante Y' des zu variierenden Proteins Y zu binden, oder eine für einen derartigen Komplex kodierende Nukleinsäuresequenz.

Weiterhin betrifft die Erfindung einen alternativen Kit zur Herstellung eines Proteins mit verbesserten Eigenschaften, umfassend
(a) eine Expressionsumgebung,
(b) eine Nukleinsäuresequenz S, kodierend für
   (i) eine durch eine RNA-Polymerase Pol *in trans* aktivierbare Transkriptionskontrollsequenz,
   (ii) ein zu variierendes Protein Y,
   (iii) eine RNA-Polymerase Pol,
(c) einen Komplex, umfassend eine Reverse Transkriptase RT und ein Protein X, welches in der Lage ist, an mindestens eine Variante Y' des zu variierenden Proteins Y zu binden, oder eine für einen derartigen Komplex kodierende Nukleinsäuresequenz.

### Beschreibung der Figur

Figur 1 zeigt eine schematische Darstellung eines Verfahrens gemäß Anspruch 1. In einer mit einem NASBA-Reaktionsgemisch gefüllten Kapillarsystem verläuft eine Polymerisations- und Evolutionsfront. Hier werden räumlich bevorzugt Moleküle erzeugt, welche für Varianten kodieren, die mit dem an die T7-RNA-Polymerase gekoppelten Protein X wechselwirken können.

### Beispiel

Ein Fusionsprotein aus T7 RNA-Polymerase (NCBI Genbank, Acc. No. P00573) und Protein A (Acc. No. CAA43604) wird in *E. coli* exprimiert, affinitätschromatografisch gereinigt und auf eine Konzentration von 5 µg/ml in PBS/10 % Glycerol eingestellt. Im Anschluss wird der Anti-HIV Env Antikörper 2F5 (Hofmann-Lehmann et al., 2001; Ferrantelli et al., 2003) im Verhältnis 1:1 an die Protein A-Domäne des chimären Fusionsproteins gebunden (= RNA-Pol./2F5 Komplex).

Eine silanisierte, an beiden Enden offene Glaskapillare mit 1 mm Innendurchmesser und einer Länge von 10 cm wird mit ca. 80 µl des folgenden Reaktionsgemisches befüllt.

*E. coli in vitro* Translations-Reaktionsgemisch (Rapid Translation System RTS) von Roche, Penzberg im Verhältnis 1:1 mit PBS.
+ RNA-Pol./2F5 Komplex zu einer Endkonzentration von 0,05 µg/ml.
+ 10 mg/ml PEG4000 zur Viskositätserhöhung.
+ RT Primer und Zweitstrangprimer zu einer Endkonzentration von 2 pmol/µl.
+ dNTP Nukleotide zu einer Endkonzentration von je 0,1 nmol/µl
+ NTP Nukleotide zu einer Endkonzentration von je 0,1 nmol/µl
+ ggf. Ethidiumbromid zu einer Endkonzentration von 0,1 ng/µl.

Die befüllte Kapillare wird in einer auf 37 °C beheizten Kammer waagrecht befestigt und an einem Ende mit 0,5 µl einer 1 µM Lösung eines doppelsträngigen DNA-Moleküls angeimpft. Dieses DNA-Molekül enthält den offenen Leserahmen für das Fusionsprotein aus HIV-Env und Reverser Transkriptase des Moloney Murine Leukemia Virus (Acc. No. AAO46154).
Die Reaktionskammer wird geschlossen und die Replikation der angeimpften DNA kann sich als Polymerisationsfront alternierend als Transkript (RNA) und reversern Transkript (DNA) entlang der Kapillare in Richtung vorhandener Reagenzien (zum anderen Ende) fortpflanzen.

Wurde dem Gemisch Ethidiumbromid zugesetzt, kann mittels einer UV Handlampe stündlich die Position der momentanen Polymerisationsfront festgestellt und der Endpunkt der Reaktion (Erreichen des Endes der Kapillare) bestimmt werden.
Nach Erreichen des Kapillarendes werden 2 µl des Polymerisationsfront (RNA & DNA) enthaltenden Reaktionsgemischs aus der Kapillare entnommen und die enthaltene DNA über eine PCR Reaktion mit spezifischen Oligonukleotiden amplifiziert. Das PCR Produkt wird in einen geeigneten Vektor subkloniert und in *E. coli* transformiert.

Die Sequenzauswertung von 384 Klonen ergibt eine statistische Verteilung unterschiedlicher Sequenzen, welche für evolvierte HIV-Env Varianten kodieren, die eine erhöhte Affinität zum Antikörper 2F5 aufweisen. Nähere Untersuchungen zeigen wiederkehrende Protein Motive innerhalb der Varianten, welche für die erhöhte Affinität verantwortlich sind.

### Referenzen

(1) Bauer, G.J., J.S. McCasKill and H. Otten. 1989. Traveling waves of in vitro evolving RNA. Proc.Natl.Acad.Sci.USA. 86:7937-7941
(2) Brakmann, S. and S. Grzeszik. 2001. An Error-Prone T7 RNA Polymerase Mutant Generated by Directed Evolution.. ChemBioChem. 2:212-219.
(3) Kukarin, A., M. Rong and W.T. McAllister. 2003. Exposure of T7 RNA polymerase to the isolated binding region of the promoter allows transcription from a single-stranded template. J Biol Chem. 278:2419-2424.
(4) Romano, J.W., K.G. Williams, R.N. Shurtliff, C. Ginocchio and M. Kaplan. 1997. NASBA technology: isothermal RNA amplification in qualitative and quantitative diagnostics. Immunol Invest. 26:15-28.
(5) Tanese, N., M. Roth and S.P. Goff. 1985. Expression of enzymatically active reverse transcriptase in Escherichia coli. Proc.Natl.Acad.Sci.USA. 82:4944-4948.

## Patentansprüche

1. Verfahren zur Herstellung von Varianten Y' eines Proteins Y, umfassend die Schritte:
(A) Bereitstellen eines *in vitro*-Expressionssystems, umfassend
(i) eine Nukleinsäuresequenz S, die für ein zu variierendes Protein Y kodiert,
(ii) ein Zielmolekül X, welches in der Lage ist, an das Protein Y und/oder mindestens eine Variante Y' davon zu binden,
(iii) eine RNA-Polymerase (Pol), welche in der Lage ist, die Nukleinsäuresequenz S zu transkribieren,
(iv) eine Reverse Transkriptase (RT), welche in der Lage ist, Transkripte der Nukleinsäuresequenz S revers zu transkribieren,
wobei entweder das Zielmolekül X an Pol und das Protein Y an RT gekoppelt ist,
oder das Zielmolekül X an RT und das Protein Y an Pol gekoppelt ist,
(B) Inkubieren des *in vitro* Expressionssystems aus (A) unter Bedingungen, welche eine Transkription, eine Reverse Transkription und eine Translation unter Bildung von Varianten Y' des Proteins Y und dafür kodierender Nukleinsäuresequenzen S' ermöglichen, und welche die Bildung von Varianten Y' mit verbesserten Bindungseigenschaften für das Zielmolekül X begünstigen,
(C) Isolieren derjenigen Varianten Y', welche verbesserte Bindungseigenschaften für die Bindung an X aufweisen und/oder Isolieren von für Y' kodierenden Nukleinsäuresequenzvarianten S'.

2. Verfahren nach Anspruch 1, umfassend die Schritte:
(A) Bereitstellen eines in vitro-Expressionssystems, umfassend
(a) eine Nukleinsäuresequenz S, kodierend für
(a1) eine durch eine RNA-Polymerase Pol *in trans* aktivierbare Transkriptionskontrollsequenz,
(a2) ein zu variierendes Protein Y, und
(a3) eine Reverse Transkriptase (RT),
oder alternativ
(a3') eine RNA-Polymerase (Pol),
wobei die für (a2) und (a3) bzw. (a3') kodierenden Sequenzabschnitte für ein Fusionsprotein unter der Kontrolle der *in trans* aktivierbaren Transkriptionskontrollsequenz von (a1) kodieren,
(b) einen Proteinkomplex, umfassend
(b1) eine Komponente X, welche in der Lage ist, an das Protein Y und/oder mindestens eine Variante Y' des zu variierenden Proteins Y zu binden, und
(b2) die RNA-Polymerase (Pol) zur Transkription der Nukleinsäuresequenz S aus (a)
oder alternativ
(b2') die Reverse Transkriptase (RT),
(B) Inkubieren des *in vitro* Expressionssystems aus (A) unter Bedingungen, welche die Bildung von Varianten Y' des Proteins Y ermöglichen,
(C) Isolieren derjenigen Varianten Y', welche verbesserte Bindungseigenschaften für die Bindung an X aufweisen und/oder Isolieren der für Y' kodierenden Nukleinsäuresequenzvarianten S'.

3. Verfahren nach einem der vorhergehenden Ansprüche,
wobei ein Komplex aus Pol, RT, X und einer Variante Y' gebildet wird, der eine Reverse Transkription desjenigen Transkripts ermöglicht, von welchem diese Variante Y' kodiert wurde.

4. Verfahren nach einem der vorhergehenden Ansprüche,
wobei die Nukleinsäuresequenz S entweder für ein Fusionsprotein aus Y und RT oder für ein Fusionsprotein aus Y und Pol kodiert.

5. Verfahren nach einem der vorhergehenden Ansprüche,
wobei Pol, RT und/oder X von einer Nukleinsäure kodiert werden oder als Proteine oder Fusionsproteine bereitgestellt werden.

6. Verfahren nach einem der vorhergehenden Ansprüche,
wobei als *in vitro*-Expressionssystem eine Kapillare, eine zweidimensionale Expressionsumgebung oder eine dreidimensionale Expressionsumgebung verwendet wird.

7. Verfahren nach einem der vorhergehenden Ansprüche 2 bis 6,
wobei die Transkriptionskontrollsequenz ausgewählt wird aus einem RNA-Polymerase-T7-Promotor, einem RNA-Polymerase-T3-Promotor und einem RNA-Polymerase-SP6-Promotor.

8. Verfahren nach einem der vorhergehenden Ansprüche,
wobei als Pol RNA-Polymerase-T7, RNA-Polymerase-T3 oder RNA-Polymerase-SP6 verwendet wird.

9. Verfahren nach einem der vorhergehenden Ansprüche,
wobei als Pol eine RNA-Polymerase verwendet wird, die eine erhöhte Fehlerrate als die korrespondierende Wildtyp-Polymerase aufweist.

10. Kit zur Herstellung eines Proteins mit verbesserten Eigenschaften, umfassend
(a) eine Expressionsumgebung,
(b) eine Nukleinsäuresequenz S, kodierend für
(b1) eine durch eine RNA-Polymerase *in trans* aktivierbare Expressionskontrollsequenz,
(b2) ein zu variierendes Protein Y,
(b3) eine Reverse Transkriptase,
(c) einen Komplex, umfassend eine RNA-Polymerase und ein Zielmolekül X, welches in der Lage ist, an mindestens eine Variante Y' des zu variierenden Proteins Y zu binden, oder eine für einen derartigen Komplex kodierende Nukleinsäuresequenz.

11. Kit zur Herstellung eines Proteins mit verbesserten Eigenschaften, umfassend
(a) eine Expressionsumgebung,
(b) eine Nukleinsäuresequenz S, kodierend für
(b1) eine durch ein Protein P *in trans* aktivierbare Transkriptionskontrollsequenz,
(b2) ein zu variierendes Protein Y,
(b3') eine RNA-Polymerase,
(c) einen Komplex, umfassend eine Reverse Transkriptase und ein Zielmolekül X, welches in der Lage ist, an mindestens eine Variante Y' des zu variierenden Proteins Y zu binden, oder eine für einen derartigen Komplex kodierende Nukleinsäuresequenz.

12. Kit nach Anspruch 10 oder 11,
wobei die Expressionsumgebung ausgewählt ist aus einer Kapillare, einer zweidimensionalen Expressionsumgebung und einer dreidimensionalen Expressionsumgebung.

13. Kit nach einem der Ansprüche 10 bis 12,
zusätzlich umfassend geeignete-Primer, dNTPs, NTPs, und/oder Puffer.

## Claims

1. Method for producing variants Y' of a protein Y, comprising the steps:
(A) provision of an *in vitro* expression system comprising
(i) a nucleic acid sequence S which codes for a protein Y which is to be varied,
(ii) a target molecule X which is able to bind to the protein Y and/or at least one variant Y' thereof,
(iii) an RNA polymerase (Pol) which is able to transcribe the nucleic acid sequence S,
(iv) a reverse transcriptase (RT) which is capable of reverse transcription of transcripts of the nucleic acid sequence S,
where either the target molecule X is coupled to Pol and the protein Y is coupled to RT,
or the target molecule X is coupled to RT and the protein Y is coupled to Pol,
(B) incubation of the *in vitro* expression system from (A) under conditions which enable transcription, reverse transcription and translation to form variants Y' of the protein Y and nucleic acid sequences S' coding therefor, and which favor the formation of variants Y' with improved binding properties for the target molecule X,
(C) isolation of those variants Y' which exhibit improved binding properties for binding to X, and/or isolation of nucleic acid sequence variants S' coding for Y'.

2. Method according to claim 1, comprising the steps:
(A) provision of an *in vitro* expression system comprising
(a) a nucleic acid sequence S coding for
(a1) a transcription control sequence activatable *in trans* by an RNA polymerase Pol,
(a2) a protein Y to be varied, and
(a3) a reverse transcriptase (RT),
or alternatively
(a3') an RNA polymerase (Pol),
where the sequence segments coding for (a2) and (a3) or (a3') code for a fusion protein under the control of the transcription control sequence activatable *in trans* of (a1),
(b) a protein complex comprising
(b1) a component X which is able to bind to the protein Y and/or at least one variant Y' of the protein Y to be varied, and
(b2) the RNA polymerase (Pol) for transcription of the nucleic acid sequence S from (a)
or alternatively
(b2') the reverse transcriptase (RT),
(B) incubation of the *in vitro* expression system from (A) under conditions which enable the formation of variants Y' of the protein Y,
(C) isolation of those variants Y' which exhibit improved binding properties for binding to X, and/or isolation of the nucleic acid sequence variants S' coding for Y'.

3. Method according to either of the preceding claims, where a complex of Pol, RT, X and a variant Y' which enables reverse transcription of the transcript by which this variant Y' was encoded is formed.

4. Method according to any of the preceding claims,
where the nucleic acid sequence S codes either for a fusion protein composed of Y and RT or for a fusion protein composed of Y and Pol.

5. Method according to any of the preceding claims,
where Pol, RT and/or X are encoded by a nucleic acid or are provided as proteins or fusion proteins.

6. Method according to any of the preceding claims,
where a capillary, a two-dimensional expression environment or a three-dimensional expression environment is used as *in vitro* expression system.

7. Method according to any of the preceding claims 2 to 6, where the transcription control sequence is selected from an RNA polymerase T7 promoter, an RNA polymerase T3 promoter and an RNA polymerase SP6 promoter.

8. Method according to any of the preceding claims,
where RNA polymerase T7, RNA polymerase T3 or RNA polymerase SP6 is used as Pol.

9. Method according to any of the preceding claims,
where an RNA polymerase exhibiting an increased error rate than the corresponding wild-type polymerase is used as Pol.

10. Kit for producing a protein with improved properties, comprising
(a) an expression environment,
(b) a nucleic acid sequence S coding for
(b1) an expression control sequence activatable *in trans* by an RNA polymerase,
(b2) a protein Y to be varied,
(b3) a reverse transcriptase,
(c) a complex comprising an RNA polymerase and a target molecule X which is able to bind to at least one variant Y' of the protein Y to be varied, or a nucleic acid sequence coding for such a complex.

11. Kit for producing a protein with improved properties, comprising
(a) an expression environment,
(b) a nucleic acid sequence S coding for
(b1) a transcription control sequence activatable *in trans* by a protein P,
(b2) a protein Y to be varied,
(b3') an RNA polymerase,
(c) a complex comprising a reverse transcriptase and a target molecule X which is able to bind to at least one variant Y' of the protein Y to be varied, or a nucleic acid sequence coding for such a complex.

12. Kit according to claim 10 or 11, where the expression environment is selected from a capillary, a two-dimensional expression environment and a three-dimensional expression environment.

13. Kit according to any of claims 10 to 12, additionally comprising suitable primers, dNTPs, NTPs and/or buffers.

## Revendications

1. Procédé de production de variantes Y' d'une protéine Y, comprenant les étapes consistant à :
(A) se munir d'un système d'expression *in vitro*, comprenant
(i) une séquence d'acide nucléique S qui code pour une protéine soumise à variation Y,
(ii) une molécule cible W qui est capable de se lier à la protéine Y et/ou à au moins une variante Y' de celle-ci,
(iii) une ARN polymérase (Pol) qui est capable de transcrire la séquence d'acide nucléique S,
(iv) une transcriptase inverse (RT) qui est capable de transcrire inversement des produits de transcriptions de la séquence d'acide nucléique S,
la molécule cible X étant couplée à Pol et la protéine Y, à RT,
ou la molécule cible X étant couplée à RT et la protéine Y à Pol,
(B) incuber le système d'expression *in vitro* du point (A) dans des conditions qui permettent une transcription, une transcription inverse et une traduction en formant des variantes Y' de la protéine Y et des séquences d'acide nucléique S' codant pour celles-ci, et qui favorisent la formation de variantes Y' avec des propriétés de liaison améliorées pour la molécule cible X,
(C) isoler les variantes Y' qui présentent des propriétés de liaison améliorées pour la liaison à X et/ou isoler des variantes de séquences d'acide nucléique S' codant pour Y'.

2. Procédé selon la revendication 1, comprenant les étapes consistant à :
(A) se munir d'un système d'expression *in vitro,* comprenant
(a) une séquence d'acide nucléique S codant pour
(a1) une séquence de contrôle de la transcription activable en trans par une ARN polymérase Pol,
(a2) une protéine Y soumise à variation, et
(a3) une transcriptase inverse (RT),
ou en variante
(a3') une ARN polymérase (Pol),
les segments de séquences codant pour (a2) et (a3) ou (a3') codant pour une protéine de fusion sous le contrôle de la séquence de contrôle de transcription de (a1) activable en trans,
(b) un complexe de protéine, comprenant
(b1) un composant X qui est capable de se lier à la protéine Y et/ou à au moins une variante Y' de la protéine soumise à variation, et
(b2) l'ARN polymérase (Pol) pour la transcription de la séquence d'acide nucléique S du point (a)
ou en variante
(b2') la transcriptase inverse (RT),
(B) incuber le système d'expression *in vitro* du point (A) dans des conditions qui permettent la formation de variantes Y' de la protéine Y,
(C) isoler les variantes Y' qui améliorent les propriétés de liaison pour la liaison à X et/ou isoler les variantes de séquence d'acide nucléique S' codant pour Y'.

3. Procédé selon l'une quelconque des revendications précédentes,
dans lequel un complexe de Pol, RT, X et d'une variante Y' est formé, qui permet une transcription inverse du produit de transcription à partir duquel cette variante Y' a été codée.

4. Procédé selon l'une quelconque des revendications précédentes,
dans lequel la séquence d'acide nucléique S code pour une protéine de fusion de Y et RT ou pour une protéine de fusion de Y et Pol.

5. Procédé selon l'une quelconque des revendications précédentes,
dans lequel Pol, RT et/ou X sont codés par un acide nucléique ou sont mis à disposition sous forme de protéines ou protéines de fusion.

6. Procédé selon l'une quelconque des revendications précédentes,
dans lequel on utilise comme système d'expression *in vitro,* un capillaire, un environnement d'expression bidimensionnel ou un environnement d'expression tridimensionnel.

7. Procédé selon l'une quelconque des revendications 2 à 6 précédentes,
dans lequel la séquence de contrôle de la transcription est choisie à partir d'un promoteur d'ARN polymérase T7, d'un promoteur d'ARN polymérase T3 et d'un promoteur d'ARN polymérase SP6.

8. Procédé selon l'une quelconque des revendications précédentes,
dans lequel on utilise en tant que Pol, l'ARN polymérase T7, l'ARN polymérase T3
ou l'ARN polymérase SP6.

9. Procédé selon l'une quelconque des revendications précédentes,
dans lequel on utilise en tant que Pol, une ARN polymérase qui présente un taux d'erreur supérieur à celui de la polymérase de type sauvage correspondante.

10. Kit de production d'une protéine présentant des propriétés améliorées, comprenant
(a) un environnement d'expression,
(b) une séquence d'acide nucléique S, codant pour
(b1) une séquence de contrôle de l'expression activable en trans par une ARN polymérase,
(b2) une protéine Y soumise à variation,
(b3) une transcriptase inverse,
(c) un complexe comprenant une ARN polymérase et une molécule cible X qui est capable de se lier à au moins une variante Y' de la protéine Y soumise à variation, ou une séquence d'acide nucléique codant pour un complexe de ce type.

11. Kit de production d'une protéine présentant des propriétés améliorées, comprenant
(a) un environnement d'expression,
(b) une séquence d'acide nucléique S, codant pour
(b1) une séquence de contrôle de transcription activable en trans par une protéine P,
(b2) une protéine Y soumise à variation,
(b3') une ARN polymérase,
(c) un complexe comprenant une transcriptase inverse et une molécule cible X qui est capable de se lier à au moins une variante Y' de la protéine Y soumise à variation, ou à une séquence d'acide nucléique codant pour un complexe de ce type.

12. Kit selon la revendication 10 ou 11,
dans lequel l'environnement d'expression est choisi parmi un capillaire, un environnement d'expression bidimensionnel et un environnement d'expression tridimensionnel.

13. Kit selon l'une quelconque des revendications 10 à 12,
comprenant en outre des amorces appropriées, dNTPs, NTPs et/ou un tampon
